# EUROPEAN PATENT APPLICATION

(11) **EP 2 647 975 A2**
(43) Date of publication of application: **09.10.2013**
(21) Application number: 13162049.4
(22) Date of filing: 03.04.2013
(51) Int. Cl.: G01N 1/40, G01N 15/06, B01L 3/00

(54) **Filter for capturing target material**

(30) Priority: 05.04.2012 KR 20120035601
(71) Applicant: Samsung Electronics Co., Ltd, Gyeonggi-do 443-742 (KR)
(72) Inventor: Lee, June-young, Gyeonggi-do (KR); Moon, Hui-sung, Gyeonggi-do (KR); Kim, Min-seoks, Gyeonggi-do (KR); Kim, Yeon-jeong, Gyeonggi-do (KR); Baek, Sang-hyun, Gyeonggi-do (KR); Sim, Tae-seok, Gyeonggi-do (KR); Oh, Jin-mi, Gyeonggi-do (KR); Lee, Jeong-gun, Gyeonggi-do (KR); Lee, Hun-joo, Gyeonggi-do (KR); Jeong, Hyo-young, Gyeonggi-do (KR)
(74) Representative: Grünecker, Kinkeldey, Stockmair & Schwanhäusser

(57) **Abstract**

A target material capturing filter is described herein. The target material capturing filter may include an inlet through which a fluid enters; an outlet through which at least a portion of the fluid is discharged; a first flow path that is connected to the inlet; a second flow path that is connected to the outlet; and a filter unit that is disposed between the first flow path and the second flow path and captures the target material by letting at least a portion of the fluid that flows through the first flow path drop through to the second flow path.

## Description

### BACKGROUND

Early detection of cancer is vitally important, and much research has been conducted to find accurate, simple, and rapid diagnosis methods. Recently, a method of diagnosing cancer by capturing circulating tumor cells (CTCs) from blood has been proposed. However, because the concentration of CTCs in blood can be little, as low as one CTC in 10⁹ cells, capturing a CTC is very difficult. For example, in the case of breast cancer, approximately less than 5 CTCs may be found in approximately 7.5 ml of blood. In the case of bowel cancer, less than about 3 CTCs may be found in approximately 7.5 ml of blood. In order to have an accurate cancer diagnosis and since the concentration of CTCs in the blood is low, it is important to capture CTCs without loss. Moreover, because CTCs are easily destroyed, capturing CTCs must be carried out by minimizing the generation of an atmosphere that adversely affects the blood cells.

For capturing CTCs, a target material capturing filter that filters the CTCs and allows flowing, for example, of white blood cells and red blood cells, may be used. The target material capturing filter may have a structure in which column-type patterns are formed in a fine flow channel through which blood may typically flow. White blood cells and red blood cells, which have relatively a small size, may pass through the patterns, while CTCs, which have a relatively large size, may be captured between the patterns. However, in the target material capturing filter having the above-described structure, flow paths may be clogged by the captured CTCs. When the flow paths are clogged, stress is applied to the CTCs, which may damage the CTCs. Also, white blood cells are captured together with CTCs, which results in the reduction of analysis efficiency and in an increase in the analyzing time.

### SUMMARY

Provided herein are target material capturing filters that safely capture a target material in a fluid by making the fluid flow in a three-dimensional flow.

Additional aspects will be set forth in part in the description which follows and, in part, will be apparent from the description, or may be learned by practice of the presented embodiments.

According to an aspect of the present invention, there is provided a target material capturing filter including: an inlet through which a fluid enters; an outlet through which at least a portion of the fluid is discharged; a first flow path that is connected to the inlet; a second flow path that is connected to the outlet; and a filter unit that is disposed between the first flow path and the second flow path and captures the target material by allowing at least a portion of the fluid that flows through the first flow path to drop through the filter unit to the second flow path, while the target material is retained in the filter unit. This facilitates a change in direction of the fluid flowing through the first flow path as it enters the filter unit, so that the target material may be more easily captured.

The flow direction of the fluid that flows through the first flow path and the flow direction of the fluid that flows through the second flow path may be substantially parallel to each other. The flow direction of the fluid that flows through the first flow path and the flow direction of the fluid that flows through the second flow path may be parallel to each other by separating a distance greater than a thickness of the filter unit disposed between the first flow path and the second flow path. Due to the separation, the flow paths may be parallel to each other, which may allow a flat design of the target material capturing filter.

The flow direction of a fluid that flows through the first flow path may be substantially perpendicular to the flow direction of the fluid that drops through the filter unit. Accordingly, the flow path through the filter unit may be substantially perpendicular to the dropping direction. For example, the dropping direction may be perpendicular to both, the flow direction of the fluid that flows through the first flow path and the flow direction of the fluid that flows through the second flow path.

The filter unit may include at least one opening through which fluid from the first flow path passes to the second flow path. The filter unit may be a substrate having at least one opening, and the substrate may have any suitable shape such as the shape of a plate shape or a film or a bar shape. A length of the opening, that is, a dimension of the opening in a direction of the first flow path or the second flow path (e.g., the general direction of flow from the inlet to the outlet) may be greater than a width of the opening, that is, a dimension of the opening in a direction substantially perpendicular to the first flow path or the second flow path (e.g., a direction substantially perpendicular to the general direction of flow from the inlet to the outlet). A depth of the opening, that is, a dimension corresponding to the thickness of the substrate in which the filter unit is disposed, is not specifically limited as long as the filter unit is able to pass at least a portion of incoming fluid and retain a target material. For example, the depth of the second substrate comprising the filter unit may be in a range from about 5 µm to about 50 µm. Thus, the one or more openings of the filter unit may have a rectangular shape with a length (dimension in the direction of the first flow path or the second flow path) greater than the width. Also, when the filter unit comprises a plurality of openings, the openings may be arranged as one-dimensional type array or a two-dimensional type array. The one-dimensional type array may be, for example, a single row of openings in a direction substantially perpendicular to the general direction of flow from the inlet to the outlet. The two-dimensional type array may be, for example, an arrangement of the openings in multiple such rows. These features have the advantage that an excessive increase of the fluid speed may be prevented and a pressure being applied to the filter unit may be reduced.

The one or more openings may have any suitable shape, such as a polygonal shape, a circular shape, or an oval shape. When the filter unit comprises multiple openings, the openings can each have shapes that are the same or different from one another. Such opening shapes are particularly easy to manufacture.

The at least one opening may have a width through which materials other than the target material may pass. The at least one opening may have a width smaller than the diameter of the target material, and may have a length greater than the diameter of the target material. Accordingly, the target material may loose speed and accumulate. However, even though the target material may be clogged in some regions of the filter unit, the opening provides sufficient room for passing the fluid. Accordingly, the filter unit is not completely clogged. Since there is little flow speed and flow pressure of the fluid in regions where the target material is clogged, there is no loss of captured target material due to flow speed and flow pressure of the fluid.

The target material may accumulate in a predetermined region of the filter unit, such as a region of the filter unit where the speed of the fluid containing the target material is reduced. Accordingly, the filter unit may capture the target material.

The target material capturing filter may further include a fluid resistance unit that is disposed in the first flow path and controls the fluid that flows through the first flow path. The fluid resistance unit may have a lozenge shape (e.g., a diamond shape). The fluid resistance unit may be provided by a protrusion into the first flow path. This allows for a good control of the fluid speed and/or adjustment of the filtering properties.

According to another aspect of the present invention, there is provided a target material capturing filter including: a first substrate; a third substrate that is separated from the first substrate; a second substrate disposed between the first and third substrates and in contact with a lower surface of the first substrate and an uppersurface of the third substrate; a first flow path formed by etching the lower surface of the first substrate; a second flow path formed by etching an upper surface of the third substrate; and a filter unit that is formed by penetrating through the second substrate, captures the target material in a fluid that flows through the first flow path, and allows a portion of the fluid to flow through the second flow path. This provide for an easy way of manufacturing the target material capturing filter. The advantages of the following developments generally correspond to the advantages already given above.

The target material capturing filter may further include an inlet that contacts with, and is in fluid communication with the first flow path by penetrating through the first substrate.

The target material capturing filter may further include an outlet that contacts with, and is in fluid communication with the second flow path by penetrating through the first substrate and the second substrate. The target material capturing filter may further include an outlet that contacts the second flow path by penetrating through at least a portion of the third substrate.

The filter unit may include at least one opening, and the at least one opening may have any suitable shape, such as a polygonal shape, a circular shape, and an oval shape. The filter unit can have a plurality of openings, each of which may have a shape that is the same or different from that of the other openings.

The at least one opening may have a width smaller than the diameter of the target material, and may have a length greater than the diameter of the target material.

The target material may accumulate in a predetermined region of the filter unit, such as a region of the filter unit where the speed of the fluid containing the target material is reduced.

The target material capturing filter may minimize a pressure to be applied to the filter unit by differentiating the flow direction of the fluid that flows through the filter unit and the flow direction of the target material that is captured in the filter unit. Accordingly, because the pressure change is minimized, damage to the captured target material is prevented. Also, because the target material is captured in a predetermined region of the filter unit, the analysis of the target material is easy.

### BRIEF DESCRIPTION OF THE DRAWINGS

These and/or other aspects will become apparent and more readily appreciated from the following description of the embodiments, taken in conjunction with the accompanying drawings in which:

FIG. 1 is a schematic perspective view of a target material capturing filter according to an embodiment of the present invention;

FIG. 2 is a schematic exploded perspective view of the target material capturing filter of FIG. 1;

FIG. 3 is a schematic cross-sectional view taken along line A-A' of the target material capturing filter of FIG. 1;

FIG. 4A is a graph showing the measuring result of a flow speed of a fluid in a y-axis direction in a filter unit according to an embodiment of the present invention;

FIG. 4B is a graph showing the measuring result of a flow speed of a fluid in an x-axis direction in the filter unit according to an embodiment of the present invention;

FIG. 5 is a photo-image of a target material accumulated in a region of a filter unit according to an embodiment of the present invention;

FIGS. 6A through 6E are cross-sectional views showing a method of manufacturing a target material capturing filter, according to an embodiment of the present invention;

FIG. 7 is an exploded perspective view of a target material capturing filter according to an embodiment of the present invention; and

FIG. 8 is an exploded perspective view of a target material capturing filter according to an embodiment of the present invention.

### DETAILED DESCRIPTION

Reference will now be made in detail to embodiments, examples of which are illustrated in the accompanying drawings, wherein like reference numerals refer to like elements throughout.

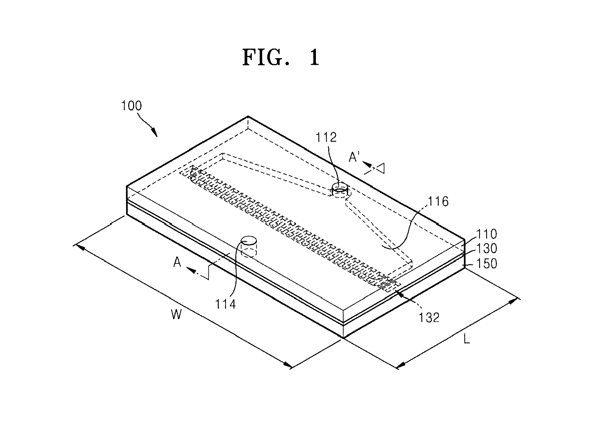

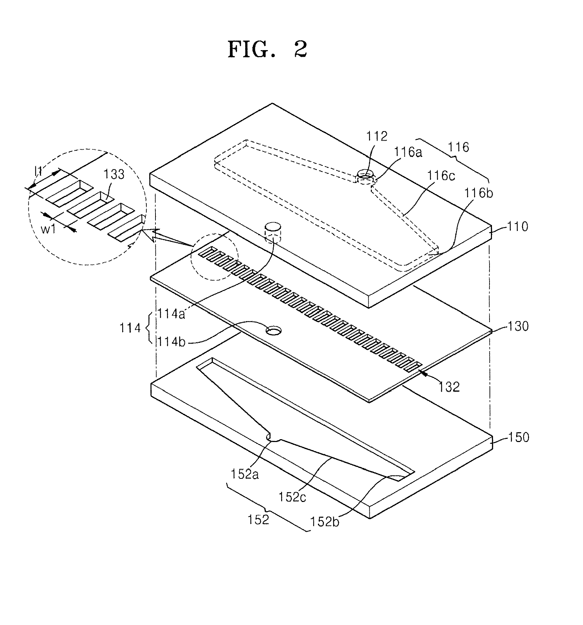

FIG. 1 is a schematic perspective view of a target material capturing filter 100 according to an embodiment of the present invention. FIG. 2 is a schematic exploded perspective view of the target material capturing filter 100 of FIG. 1, in which surface structures of three substrates, namely, first substrate 110, second substrate 130, and third substrate 150 are shown.

Referring to FIGS. 1 and 2, the target material capturing filter 100 may include an inlet 112 through which a fluid to be inspected flows in; an outlet 114 through which an inspected fluid flows out; a first flow path 116 that is connected to the inlet 112 and through which the fluid that flows in through the inlet 112 flows; a second flow path 152 that is connected to the outlet 114 and through which the fluid flows towards the outlet 114; and a filter unit 132 that is disposed between the first and second flow paths 116 and 152 and captures a target material by allowing at least a portion of the fluid flowing through the first flow path 116 to drop through the filter unit to the second flow path 152, thereby facilitating a change in direction of the fluid flowing through the first flow path 116 as it enters the filter unit 132.

A fluid that flows through the target material capturing filter 100 may have different flow directions when the fluid flows in the first flow path 116, the second flow path 152, and the filter unit 132. For example, a flow direction of a fluid that flows through the first flow path 116 may be substantially parallel to a flow direction of a fluid that flows through the second flow path 152. Parallel fluid flow in the first and second flow paths 116 and 152 may be achieved by separating the first and second flow paths 116 and 152 by a distance equal to or greater than a the thickness of the filter unit 132 disposed between the first and second flow paths 116 and 152. Also, the flow direction of the fluid that flows through the first flow path 116 and the flow direction of the fluid that changes direction to pass through the filter unit 132 may be generally perpendicular to each other. In other words, the flow path through the filter unit is substantially perpendicular to the first and second flow paths.

The target material capturing filter 100 according to an embodiment of the present invention may be formed by joining three substrates, namely, the first substrate 110, the second substrate 130, and the third substrate 150, each having a flat surface where the inlet 112, the outlet 114, the first flow path 116, the second flow path 152, and the filter unit 132 are formed.

Referring to FIG. 2, the first substrate 110 may include the inlet 112 that is formed through the first substrate 110, the first flow path 116 that is connected to the inlet 112 and is formed by etching a lower surface of the first substrate 110, and a portion of the outlet 114 (hereinafter, a first outlet 114a) that is separated from the inlet 112 and is formed through the first substrate 110. The first substrate 110 may have a rectangular shape in which the width (W) is at least twice the size of the length (L), wherein the width is the dimension generally perpendicular to the direction of flow from the outlet to the inlet, and length is the dimension generally parallel to the direction of flow from the inlet to the outlet For example, the first substrate 110 may have a width (W) of approximately 3 cm and a length (L) of approximately 1.5 cm.

The first substrate 110 may comprise transparent glass or transparent plastic, but is not limited thereto. For example, the first substrate 110 may comprise at least one of acrylate, polymethylacrylate, polymethyl methacrylate (PMMA), polycarbonate, polystyrene, polyimide, epoxy resin, polydimethylsiloxane (PDMS), and parylene.

The second substrate 130 may include the filter unit 132 that captures a target material in a fluid that flows through the first flow path 116 and passes the remaining fluid. The filter unit 132 may include at least one opening 133 that penetrates through the second substrate 130. The filter unit 132 may be the second substrate 130 having at least one opening 133. The second substrate 130 may have any suitable shape, such as the shape of a plate or a film. A length (11) of the opening 133 (that is, the dimension of the opening 133 in a direction of fluid flow in the first flow path 116 or the second flow path 152) may be greater than a width (w1) of the opening 133 (that is, the dimension of the opening 133 in a perpendicular direction to fluid flow in the first flow path 116 or the second flow path 152). A depth of the opening 133, which corresponds to the thickness of the second substrate 130, is not specifically limited as long as the depth allows passage of fluid and retention of the target material. For example, the thickness of the second substrate 130 and the corresponding depth of the opening 133 may be in a range from about 5 µm to about 50 µm. Additionally, the opening 133 may have a rectangular shape with a narrow width in the direction of the first and second flow paths 116 and 152.

In FIG. 2, the opening 133 has a rectangular shape. However, the shape of the opening 133 is not limited thereto. The opening can have any suitable shape, such as a polygonal shape, circular shape, or oval shape. The opening 133 may have a width smaller than a diameter of a target material and that may allow passing of the remaining portion of a fluid except the target material. The target material may not pass through the opening 133, and thus, may accumulate on the opening 133. Also, the opening 133 may have a length greater than the diameter of a target material. In this case, since the length of the opening 133 is greater than the diameter of the target material, clogging of the filter unit 132 by the target material may be avoided. For example, the width of the opening 133 may be in a range from about a few µm to about a few hundreds of µm, and the length of the opening 133 may be a range from about a few tens of µm to about a few mm.

In some embodiments, the filter unit 132 comprises a plurality of openings 133 arranged in a one-dimensional type array or a two-dimensional type array. In the one-dimensional type array, the openings 133 having a rectangular shape with a narrow width are arranged in parallel in a row generally perpendicular to the direction of the first flow path 116 or the second flow path 152. In the two-dimensional type array, multiple such rows are arranged. In other words, the openings 133 having a rectangular shape with a narrow width are arranged in parallel to form at least two rows, wherein each row is generally perpendicular to the direction of flow in the first or second flow paths, and the rows are repeated in the same direction of flow of the first flow path 116 or the second flow path 152.

Also, the second substrate 130 may include another portion of the outlet 114 (hereinafter, a second outlet 114b) that is separated from the filter unit 132 (e.g., in a region of the second substrate separated from the filter unit and in alignment with the outlet of the first substrate). The second outlet is formed through the second substrate 130. The second substrate 130 may have a width (W) and a length (L) that are the same as those of the first substrate 110. However, the width (W) and the length (L) of the second substrate 130 are not limited thereto. That is, the second substrate 130 may have a width and a length that are different from those of the first substrate 110. The outlet of the first substrate and the second outlet of the second substrate are arranged to form a single flow path.

The second substrate 130 may be formed of, for example,at least one of glass, quartz, transparent plastic, polymer, silicon, polysiloxane, polyurethanes, polysilicon-polyurethane, rubber, ethylene-vinyl acetate copolymer, phenolic nitrile rubber, styrene butadiene rubber, polyether-block-amides, and polyolefin.

The third substrate 150 may include the second flow path 152 formed by etching an upper surface of the third substrate 150. An end of the second flow path 152 may be connected to the filter unit 132 and the other end of the second flow path 152 may be connected to the second outlet 114b. The third substrate 150 may also have a width (W) and a length (L) that are the same as those of the first substrate 110. However, if necessary, the third substrate 150 may have a width and a length that are different from those of the first substrate 110. The third substrate 150 may be formed of a transparent glass, quartz, plastic, polymer, or the like so that captured cells or particles are observed.

The flow paths in the first and third substrates may be defined by edges. The first flow path 116 may include a first edge 116a defining a region of the first flow path 116 connected to the inlet 112, a second edge 116b defining a region of the first flow path 116 connected to the filter unit 132, and a first central edge 116c defining a region between the first and second edges 116a and 116b of the first flow path 116. As depicted in FIG. 2, the first edge 116a and the second edge 116b may have a shape corresponding to the inlet 112 and the filter unit 132, respectively. Thus, the first edge may surround at least a portion of the inlet, and the second edge may surround at least a portion of the filter unit. The first central edge 116c may be formed in a tapered shape. For example, the first central edge 116c may have a width gradually increasing towards the second edge 116b from the first edge 116a. The centeral edges, thus, define a flow path that widens from the inlet to the filter unit.For example, the first central unit 116c and flow path thereby defined may have a width that is greatly wider than a length. Also, a ratio of the width to the length of the second edge 116b or the flowpath thereby defined may be 3:1 or more and may be less than 100:1 as measured at its maximum width, width being the dimension generally perpendicular to the general direction of flow from the inlet to the outlet. In this range of ratios, an excessive increase of the fluid speed may be prevented and a pressure being applied to the filter unit 132 may be reduced.

The second flow path 152 may include a third edge 152a which defines a region of the second flow path 152 connected to the outlet 114, a fourth edge 152b which defines a region of the second flow path 152 connected to the filter unit 132, and a second central edge 152c which defines a region of the second flow path between the third and fourth edges 152a and 152b. As depicted in FIG. 2, the third edge 152a and the fourth edge 152b may have a shape generally corresponding to the inlet 112 and the filter unit 132, respectively. The second central edge 152c may be formed in a tapered shape, so as to define a flow path that narrows from the filter unit to the outlet. For example, the second central edge 152c may define a flow path having a width gradually reducing towards the fourth edge 152b from the third edge 152a. Therefore, the fluid may be smoothly discharged through the outlet 114.

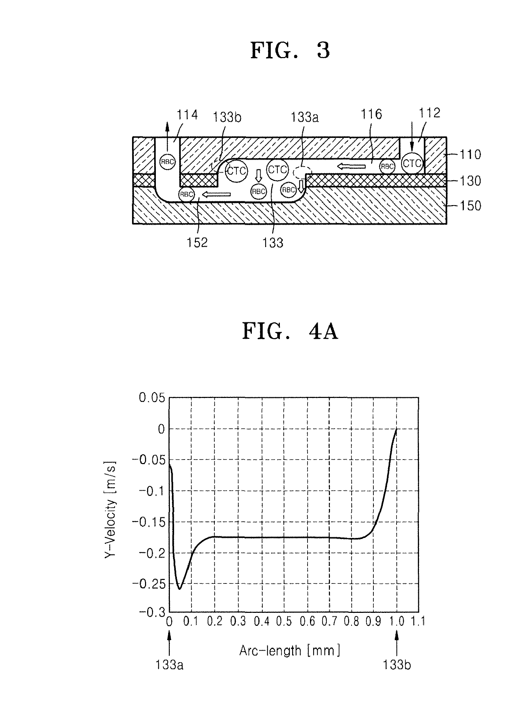

FIG. 3 is a schematic cross-sectional view taken along line A-A' of the target material capturing filter 100 of FIG. 1. An operation of the target material capturing filter 100 having the structure described above will now be described with reference to FIG. 3. For example, when a fluid flows in through the inlet 112, the fluid flows towards the outlet 114 along the first flow path 116, the filter unit 132, and the second flow path 152. Here, the fluid may be a liquid, such as blood. A height of the first flow path 116, that is, a gap between a surface formed by etching the lower surface of the first substrate 110 and an upper surface of the second substrate 130 may be a size through which the fluid may smoothly flow. For example, the first flow path 116 may have a height of approximately 50 µm. Therefore, the lower surface of the first substrate 110 may be etched to a depth of 50 µm.

The fluid that flows in a length direction of the target material capturing filter 100 may enter an upper part of the filter unit 132. At this point, fluid and materials having a size smaller than the width of the opening 133 drop to the second flow path 152 through the opening 133. For example, the flow direction of the fluid that flows through the first flow path 116 and the flow direction of the fluid that drops through the filter unit 132 may be perpendicular to each other.

The flow direction of the fluid that flows through the first flow path 116 and the flow direction of the fluid that flows through the second flow path 152 may be generally parallel to each other.

A target material that has a size greater than that of the width of the opening 133 is filtered by the filter unit 132. For example, when the opening 133 has a width of 10 µm, since red blood cells in blood fluid have a plate shape having a diameter of approximately 7-8 µm and a thickness of approximately 1-2 µm, the red blood cells may pass through the filter unit 132, that is, the opening 133. However, CTCs that have a diameter of approximately 20 µm, which is greater than the width of the opening 133, may not pass through the opening 133, and thus, may be captured by the filter unit 132.

The speed of the fluid on the filter unit 132 may differ according to the positions of the filter unit 132.

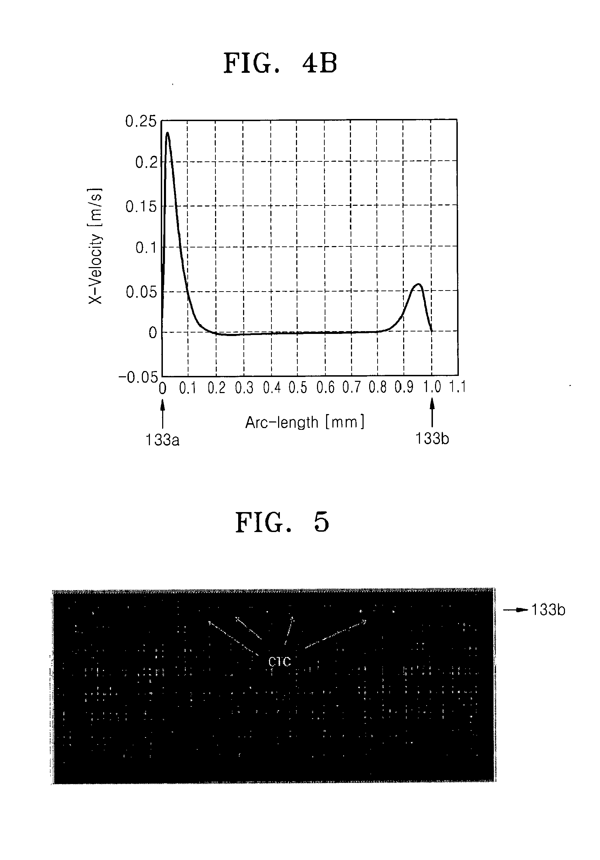

FIG. 4A is a graph showing the measuring result of flow speeds of a fluid in a y-axis direction in the filter unit 132 and FIG. 4B is a graph showing the measuring result of flow speeds of a fluid in an x-axis direction in the filter unit 132 according to an embodiment of the present invention. The "y-axis direction" is the direction of flow perpendicular to the first flow path (e.g., dropping through the filter unit). Since the y-axis direction is negative (dropping through the filter), a negative magnitude in FIG 4A represents greater velocity. The "x-axis direction" is the direction of flow parallel to the first flow path.

In FIGS. 4A and 4B, the x-axis represents a position along the length of the opening 133 in a direction from the inlet 112 to the outlet 114, and the y-axis represents flow speed of a fluid. For example, an edge portion of the opening 133 where a fluid that flows in through the inlet 112 first meets with the opening 133 is referred to as a first region 133a and the other edge portion of the opening 133 that is opposite the first region 133a, and furthest from the inlet, is referred to as a second region 133b. As depicted in FIG. 4A, the flow speed of the fluid is greatest near the first region 133a and decreases rapidly towards the second region 133b. A steady speed is maintained in a central region of the opening 133. Afterwards, the flow speed is rapidly reduced towards the second region 133b and becomes zero at the second region 133b. Also, as depicted in FIG. 4B, the flow speed is greatest near the first region 133a and decreases towards the second region 133b. The flow speed is nearly zero in the central region of the opening 133. The flow speed of the fluid increases a little near the second region 133b, and then, is reduced again.

From FIGS. 4A and 4B, it is seen that a fluid receives a maximum force in a direction from the first region 133a of the opening 133 to the second flow path 152 The target material having a size greater than the width of the opening 133 moves from the first region 133a to the second region 133b and, therefore, does not receive much force. Accordingly, the target material loses speed and accumulates in the second region 133b.

Also, the opening 133 has a length much greater than the diameter of the target material. Therefore, even though the target material is clogged in some regions of the filter unit 132, the opening 133 provides a sufficient room for passing the fluid. Accordingly, the filter unit 132 is not completely clogged.

Also, since there is little flow speed and flow pressure of the fluid in regions where the target material is clogged, there is no loss of captured target material due to flow speed and flow pressure of the fluid.

Also, since most of the fluids pass through the filter unit 132 in the first region 133a, a continuously supplied fluid may not collide with the accumulated target material in the second region 133b. Therefore, damage to or degradation of the target material due to the fluid collision may be prevented and the recovery rate of the target material may be increased.

Also, in the target material capturing filter 100 according to an embodiment of the present invention, the target material is easily observed. That is, whether the target material is captured or not and the number of captured target materials may be observed by using a microscope along an edge of the filter unit 132 that is formed in a straight line shape. FIG. 5 is a photo-image of a target material accumulated in a region of the filter unit 132 according to an embodiment of the present invention. From FIG. 5, it is seen that the target materials captured in the filter unit 132 are accumulated in an edge region of the filter unit 132.

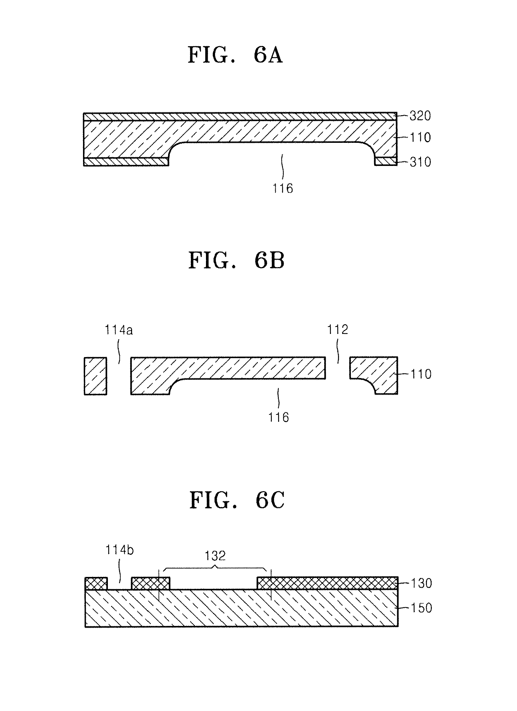

FIGS. 6A through 6E are cross-sectional views showing a method of manufacturing the target material capturing filter 100, according to an embodiment of the present invention.

As depicted in FIG. 6A, etch mask layers 310 and 320 are formed respectively on an upper surface and lower surface of the first substrate 110. The first substrate 110 may be formed of transparent glass or transparent plastic, but is not limited thereto. For example, the first substrate 110 may be one of acrylate, polymethylacrylate, PMMA, polycarbonate, polystyrene, polyimide, epoxy resin, PDMS, and parylene.

The etch mask layers 310 and 320 are patterned by using a photolithography process. The first flow path 116 is formed by wet etching the first substrate 110 using an HF etchant. When the first flow path 116 is formed, a region of the first flow path 116 where the flow direction of the fluid is changed may be formed in a curved shape to avoid damaging the target material.

Next, after removing the etch mask layers 310 and 320 from the first substrate 110, as depicted in FIG. 6B, the inlet 112 and the first outlet 114a that penetrate the first substrate 110 are formed. The inlet 112 and the first outlet 114a may be formed by using a sand blast process. The inlet 112 and the first outlet 114a may be formed separate from each other. The inlet 112 may be formed to contact with the first flow path 116 and the first outlet 114a may be formed not to contact with the first flow path 116.

Next, the second substrate 130 is formed on the third substrate 150. The second substrate 130 may one of silicon, polysiloxane, polyurethanes, polysilicon-polyurethane, rubber, ethylene-vinyl acetate copolymer, phenolic nitrile rubber, styrene butadiene rubber, polyether-block-amides, and polyolefin. The third substrate 150 and the first substrate 110 may be one of glass, acrylate, polymethylacrylate, PMMA, polycarbonate, polystyrene, polyimide, epoxy resin, PDMS, and parylene. Also, the second substrate 130 and the third substrate 150 may be silicon-on-glass (SOG).

As depicted in FIG. 6C, the second outlet 114b and the filter unit 132 that penetrate the second substrate 130 may be formed. An SOG wafer may be patterned by using, for example, a photolithography process. Next, the second outlet 114b and the filter unit 132 are formed in a silicon layer by using a deep reactive ion etching (DRIE) process. The filter unit 132 may include at least one opening 133. The opening 133 may have a width smaller than a diameter of a target material so that the target material may not pass through the opening 133. However, a length of the opening 133 may be greater than the diameter of the target material. Additionally, the at least one opening 133 may be formed as a one-dimensional type array, but is not limited thereto. The at least one opening 133 may be formed as a two-dimensional type array. The second outlet 114b may be separated from the at least one opening 133.

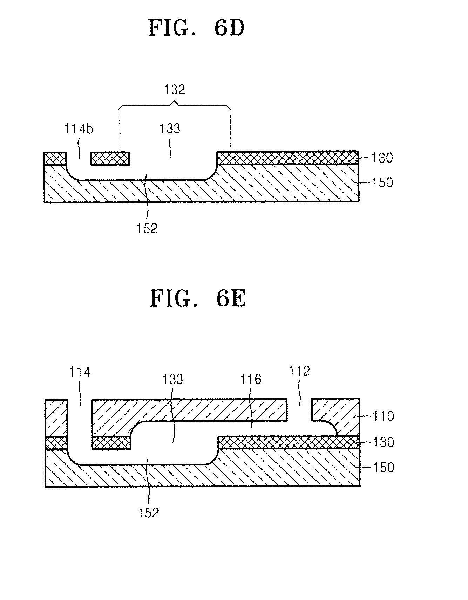

Also, as depicted in FIG. 6D, the second flow path 152 is formed by etching a portion of the third substrate 150. An end of the second flow path 152 may be connected to the filter unit 132 and the other end of the second flow path 152 may be connected to the second outlet 114b. For example, an SOG wafer on which the second outlet 114b and the filter unit 132 are formed is etched by using an HF etchant.

Next, as depicted in FIG. 6E, the first substrate 110 in which the inlet 112, the first flow path 116, and the first outlet 114a are formed is combined with the second substrate 130 in which the second outlet 114b and the at least one opening 133 are formed. The first substrate 110 and the second substrate 130 may be combined by using an anodic bonding process. In this way, the target material capturing filter 100 according to an embodiment of the present invention may be readily manufactured by using an etching process.

The target material capturing filter 100 may further include an element that may control a fluid that flows in the first flow path 116.

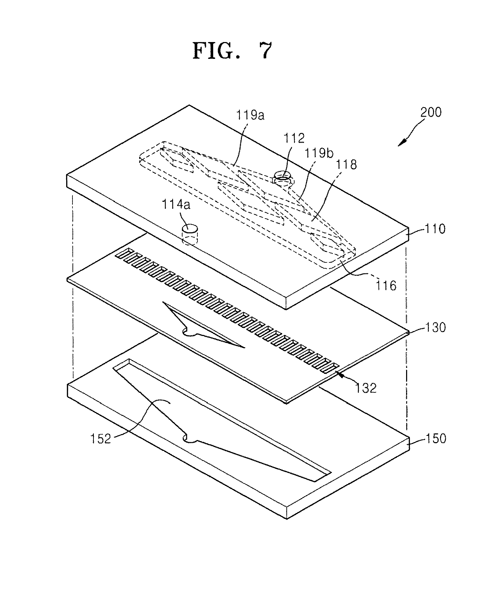

FIG. 7 is an exploded perspective view of a target material capturing filter 200 according to another embodiment of the present invention. The target material capturing filter 200 may further include a fluid resistance unit 118 that protrudes inwards of a first flow path 116. As depicted in FIG. 7, a fluid that enters through the inlet 112 flows towards an edge of the first flow path 116 through two narrow fine channels 119a and 119b which are formed between inner walls of the fluid resistance unit 118 and the first flow path 116. Afterwards, the fluid may flow from the edge to a central region of the first flow path 116.

The fluid resistance unit 118 may control a speed and a stream line of the fluid that enters through the inlet 112. For example, the fluid resistance unit 118 may reduce the speed of a fluid that enters through the inlet 112 by preventing the fluid from directly entering into the first flow path 116, and may maintain the flow speed of the fluid at a predetermined range in the first flow path 116. Also, the fluid resistance unit 118 may evenly distribute stream lines of the fluid in the first flow path 116 and may control the length of the stream lines that are similar to each other. Accordingly, the fluid may uniformly flow along the first flow path 116 by the fluid resistance unit 118 and the concentration of the fluid in a specific region in the filter unit 132 may be prevented.

As depicted in FIG. 7, the fluid resistance unit 118 may have a lozenge shape or a diamond shape, but the shape is not limited thereto. For example, the fluid resistance unit 118 may have a polygonal shape, such as a triangular shape or a rectangular shape, and also, may have a circular shape, an oval shape, a fan shape, a stream line shape, or a combination of these shapes.

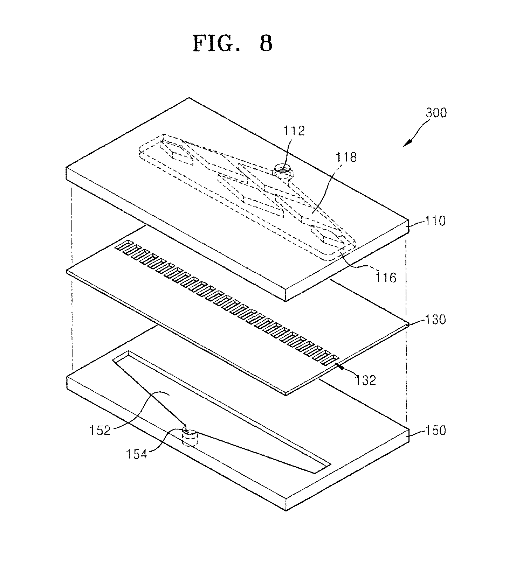

Also, in FIGS. 1 and 7, the inlet 112 and the outlet 114 are formed together in the first substrate 110. However, in another embodiment the inlet 112 and the outlet 114 may be formed in different substrates. FIG. 8 is an exploded perspective view of a target material capturing filter 300 according to another embodiment of the present invention. As depicted in FIG. 8, the inlet 112 may be formed to contact with the first flow path 116 through the first substrate 110, and the outlet 114 may be formed to contact with the second flow path 152 through the third substrate 150.

Example embodiments of the filter for capturing a target material have been particularly described and shown in the accompanying drawings. However, it should be understood that the example embodiments described herein should be considered in a descriptive sense only and not for purposes of limitation of the present invention. Also, the scope of the invention is defined not by the detailed description of the invention but by the appended claims, because various changes in form and details may be made therein without departing from the scope of the invention by those of ordinary skill in the art.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Moreover, any combination of the above-described elements in all possible variations thereof is encompassed by the invention unless otherwise indicated herein or otherwise clearly contradicted by context.

## Claims

1. A target material capturing filter comprising:
an inlet through which a fluid enters;
an outlet through which at least a portion of the fluid is discharged;
a first flow path that is connected to the inlet;
a second flow path that is connected to the outlet; and
a filter unit that is disposed between the first flow path and the second flow path and configured to allow at least a portion of the fluid that flows through the first flow path to drop through to the second flow path, wherein target material in the fluid is captured in the filter unit.

2. The target material capturing filter of claim 1, wherein the first flow path and the second flow path are substantially parallel to each other, and/or
wherein the first flow path is perpendicular to a flow path through the filter unit.

3. The target material capturing filter of claim 1 or 2, wherein the filter unit comprises at least one opening,
in particular, wherein the filter unit comprises a plurality of openings disposed in a one-dimensional array or a two-dimensional array.

4. The target material capturing filter of claim 3, wherein the at least one opening has at least one of a polygonal shape, a circular shape, and an oval shape.

5. The target material capturing filter of claim 3 or 4, wherein the at least one opening has a width smaller than a diameter of the target material, and/or
wherein the at least one opening has a length greater than a diameter of the target material.

6. The target material capturing filter of any one of claims 1 to 5, wherein the filter unit comprises a predetermined region for accumulation of target material,
in particular, wherein the filter unit comprises a region where the speed of the target material is reduced.

7. The target material capturing filter of any one of claims 1 to 6, further comprising a fluid resistance unit that is disposed in the first flow path and controls fluid flow through the first flow path,
in particular, wherein the fluid resistance unit has at least one of a lozenge shape and a diamond shape,
preferably, wherein the fluid resistance unit is a protrusion in the first flow path.

8. A target material capturing filter according to any one of claims 1 to 7, further comprising:
a first substrate;
a third substrate that is separated from the first substrate;
a second substrate disposed between the first and third substrates and in contact with a lower surface of the first substrate and an upper surface of the third substrate;
wherein the first flow path is disposed in the lower surface of the first substrate;
the second flow path is disposed in an upper surface of the third substrate; and
the filter unit disposed in and penetrating the second substrate.

9. The target material capturing filter of claim 8, wherein the inlet is penetrating through the first substrate and in fluid communication with the first flow path.

10. The target material capturing filter of claim 8 or 9, wherein the outlet is penetrating through the first substrate and the second substrate, and in fluid communication with the second flow path, or
wherein the outlet is penetrating through the second substrate and in fluid communication with the second flow path.

11. Use of a target material capturing filter according to any one of claims 1 to 10, comprising flowing a fluid into the inlet for capturing a target material.

12. A method of manufacturing a target material capturing filter, comprising:
forming a first flow path in a lower surface of a first substrate, and an inlet and outlet separated from one another and penetrating the first substrate;
forming a filter unit in a second substrate, wherein the filter unit comprises at least one opening penetrating the second substrate;
forming a second flow path in an upper surface of a third substrate; and
disposing the second substrate between the first and third substrates, wherein the second substrate is bonded to the lower surface of the first substrate and to the upper surface of the third substrate, such that the first flow path fluidly connects the inlet and the filter unit, and the second flow path fluidly connects the filter unit and the outlet.

13. The method of claim 12, wherein the first flow path is formed in the lower surface of the first substrate by a photolithography process.

14. The method of claim 12 or 13, wherein the second substrate is bonded to the third substrate prior to forming the flow path in the third substrate.
